# EUROPEAN PATENT APPLICATION

(11) **EP 1 795 182 A1**
(43) Date of publication of application: **13.06.2007**
(21) Application number: 05781514.4
(22) Date of filing: 30.08.2005
(51) Int. Cl.: A61K 9/127, A61K 47/34, A61K 47/44, A61P 35/00, A61P 35/02

(54) **LIPOSOME IMPROVING INTRACELLULAR DRUG DELIVERY**

(30) Priority: 31.08.2004 JP 2004252328
(71) Applicant: Astellas Pharma Inc., Tokyo 103-8411 (JP); Sonobe, Takashi, Shizuoka-shi Shizuoka 420-0857 (JP)
(72) Inventor: SADZUKA, Yasuyuki, Shizuoka-shi, Shizuoka 4200839 (JP); TAKAGI, Akira, 1038411 (JP); YAMASHITA, Noboru, 1038411 (JP); SONOBE, Takashi, Shizuoka-shi, Shizuoka 420-0857 (JP)
(74) Representative: Bates, Philip Ian
(86) International application number: PCT/JP2005/015804
(87) International publication number: WO 2006/025411

(57) **Abstract**

[Problem] To provide a drug delivery system which is retained in the blood circulation and improves pharmacological effect of the drug in the target organ and cell.

[Means for Resolution] A drug-containing liposome for use in the treatment with a liposome prepared by employing polyethylene glycol cholesteryl ether as a part of the liposome-forming agent and using said liposome-forming agent. It is possible to obtain excellent drug effect by further employing a polyethylene glycol diacylglycerol ester as a part of the liposome-forming agent.

## Description

### Technical Field

This invention relates to a formulation for improving delivery of drugs into target cells, which comprises a liposome prepared using polyethylene glycol cholesteryl ether as a part of the liposome-forming agent, as well as to the liposome. Also, the present invention relates to a formulation for improving delivery of drugs into target cells, which comprises a liposome prepared by further adding a polyethylene glycol distearyl ether as a part of the liposome-forming agent, and the liposome.

### Background of the Invention

A stealth liposome which is retained in the blood circulation has been reported for the purpose of sustaining a physiologically active substance or a therapeutic compound in the body or of improving distribution of a drug, particularly an anticancer agent or antibacterial agent, in a target organ (Non-patent Reference 1), (Non-patent Reference 2) and (Non-patent Reference 3). The stealth liposome means a liposome which avoids interaction between the liposome and blood components, blood cells and vascular endothelial cells, effected by configuration of polyethylene glycol (PEG) or the like water-soluble high polymer on the liposome surface, and a PEG-modified liposome which is retained in the blood circulation is an embodiment thereof. It has been reported that such a PEG-modified liposome which shows blood-retention has a characteristic in that it is ready to be leaked and accumulated from blood into a target tissue such as a cancer tissue (Non-patent Reference 4).

A doxorubicin-encapsulated PEG-liposome which uses the aforementioned characteristic has already been put on the market in Europe and America for Kaposi sarcoma and breast cancer as the indications, and its usefulness has been reported from the viewpoint of the reduction of side effects and therapeutic effect (Non-patent Reference 5). However, though its blood retention is maintained according to said techniques, its access, contact or the like to or with the organ or cell upon which the drug acts is inhibited instead because the presence of the water-soluble high polymer becomes a barrier, so that a phenomenon in which drug concentration at the site of action does not increase, a phenomenon that the drug action is not enhanced in spite of the large amount of liposome accumulation, and the like phenomena of compromising delivery of the drug into the cells have been found as a result. For example, it has been reported in a study using tumor bearing mice that accumulation of a drug-encapsulated PEG-modified liposome and its antitumor effect are not always correlated, namely the antitumor effect is not always high when its accumulation in the tumor is high (Non-patent Reference 6).

Under such a situation, several reports have been published on the means for increasing drug effect of a drug encapsulated in liposome in a target organ or cell while keeping its retention in the circulating blood. As one of them, a method has been reported in which a water-soluble high polymer or the like efficiently acts at the time of administration, and the water-soluble high polymer is released at the adjacent of the target cell. For example, Patent Reference 1 discloses a liposome which has, on its surface, an affinity ligand effective for specifically binding to a biological surface and a hydrophilic high polymer chain effective for blocking this affinity ligand.

According to this method, the hydrophilic high polymer chain on the liposome surface effectively functions until its retention in the blood circulation is accomplished, and then the hydrophilic high polymer chain is released by the administration of a releasing agent for releasing the hydrophilic high polymer chain, so that the specific binding with the cell surface is exerted through the activation of the blocked affinity ligand. However, this method requires administration of cysteine, ascorbate or the like releasing agent for releasing the hydrophilic high polymer (chain), so that its actual use poses a problem in terms of the influence of the releasing agent in the living body and safety of the releasing agent.

Also, Patent Reference 2 discloses a technique which can change properties of a liposome through periodical release of a water-soluble high polymer from the liposome surface after its administration to the living body, without depending on the biological differences. In addition, Non-patent Reference 7 discloses a PEG-modified fusogenic liposome which can control its membrane fusion rate with cell membrane of the target cell, by changing alkyl chain length of the lipid moiety of a conjugate of polyethylene glycol and a lipid (a lipid which forms the liposome). However, these techniques have a possibility in that the retention in the circulating blood cannot be maintained due to release of the water-soluble high polymer before the liposome reaches the disease site, so that there is still room for further improvement.

On the other hand, Non-patent Reference 8 and Non-patent Reference 9 discloses a polyethylene glycol cholesteryl ether-aided liposome as a drug carrier, for the purpose of avoiding trap of the liposome by phagocyte in the liver and spleen (RES system). Said liposome showed its retention in the circulating blood by avoiding the RES system after its intravenous administration to rats. However, there is no description or suggestion in each of the aforementioned references that it is a liposome-forming agent having a feature of achieving improvement of the delivery to cells by polyethylene glycol cholesteryl ether or the ability of polyethylene glycol cholesteryl ether to improve the delivery to cells.

Patent Reference 1: JP-T-2002-503396 (the term "JP-T" as used herein means a published Japanese translation of a PCT patent application)
Patent Reference 2: JP-A-10-287554
Non-patent Reference 1: T.M. Allen and A. Chonn, "Large unilamellar liposomes with low uptake into the reticuloendothelial system", FEBS Letter, 1987, vol. 223, p. 42 - 46
Non-patent Reference 2: Alexander L. Klibanov, Kazuo Maruyama, Vladimir P. Torchilin and Leaf Huang, "Amphipathic polyethylene glycols effectively prolong the circulation time of liposomes", FEBS Letter, 1990, vol. 268, p. 235 - 237
Non-patent Reference 3: H. Takeuchi, H. Kojima, H. Yamamoto and Y. Kawasgima, "Shuju No Bubun Sosuika Suiyosei Polymer Niyoru Liposome No Hyomen Shushoku (Coating) To Sono Kettsu Tairyusei No Hyoka (Surface Modification (Coating) of Liposomes with Various Partially Hydrophobic Water-soluble Polymers and Evaluation of Their Retention in Blood)", Yakuzaigaku (Pharmacology), 2001, vol. 61, p. 86 - 96
Non-patent Reference 4: Yokoyama M. and Okano T., "Targeting of anti-cancer drugs with nano-sized carrier system", Nippon Rinsho, Japanese Journal of Clinical Medicine, 1998, vol. 56, p. 3227 - 34
Non-patent Reference 5: Coukell AJ., Spencer CM., "Polyethylene glycol-liposomal doxorubicin. A review of its pharmacodynamic and pharmacokinetic properties, and therapeutic efficacy in the management of AIDS-related Kaposi's sarcoma", Drugs, 1997, vol. 53, p. 520 - 38
Non-patent Reference 6: Hong RL., Huang CJ., Tseng YL., Pang VF., Chen ST., Chang FH., ≡Direct comparison of liposomal doxorubicin with or without polyethylene glycol coating in C-26 tumor-bearing mice: is sutface coating with polyethylene glycol beneficial?≡, Clinical Cancer Research, 1999, vol. 5, p. 3645 - 52
Non-patent Reference 7: Gitanjali Adlakha-Hutcheon, Marcel B. Bally, Clifford K. Shew & Thomas D. Madden, "Controlled destabilization of a liposomal drug delivery system enhances mitoxantrone antitumor activity", Nature Biotechnology, 1999, vol. 17, p. 775 - 779
Non-patent Reference 8: Hideaki ISHIWATA, Aline Vertut-Doi, Takeo HIROSE and Koichiro MIYAJIMA, "Physical-Chemistry Characteristics and Biodistribution of Poly(ethylene glycol)-Coated Liposomes Using Poly(oxyethylene) Cholesteryl Ether", Chem. Pharm. Bull., 1995, 43(6), p. 1005 - 1011
Non-patent Reference 9: Hideaki ISHIWATA, Satoshi B. SATO, Satoe KOBAYASHI, Masahide OKU, Aline Vertut-Doi and Koichiro MIYAJIMA, "Poly(ethylene glycol) Derivative of Cholesterol Reduces Binding Step of Liposome Uptake by Murine Macrophage-like Cell Line J774 and Human Hepatoma Cell Line HepG2", Chem. Pharm. Bull., 1998, 46(12), p. 1907 - 1913

### Disclosure of the Invention

### Problems that the invention is to solve

The challenge of the present invention is to provide a liposome which is excellent in its retention in the blood circulation and delivery into target cells, without requiring a special operation such as administration of a releasing agent, and an agent for improving delivery of a drug into a target cell, which consists of said liposome.

### Means for solving the problems

Under such a situation, the present inventors have conducted extensive studies on the method for improving delivery of a drug encapsulated in a liposome into a target organ or cell, and found as a result that polyethylene glycol cholesteryl ether, whose action has so far been revealed only about its retention in the circulating blood, has the effect to improve drug efficacy in the target organ and cell. In addition, it was found that a liposome which further contains a polyethylene glycol diacylglycerol ester as one of the lipid membrane constituting components, in addition to the polyethylene glycol cholesteryl ether, shows its retention in the circulating blood in the same manner and, to our surprise, shows higher effect than the case of direct addition of an aqueous solution of the drug to the target cell, thus resulting in the accomplishment of the present invention.

According to the present invention, it becomes possible to maintain retention of the liposome in the blood circulation and also to improve drug efficacy of a drug in the target organ or cell.

That is, the present invention relates to,
1. a formulation for improving delivery of a drug into a target cell, which comprises a liposome prepared using polyethylene glycol cholesteryl ether as a part of the liposome-forming agent,
2. the formulation for improving delivery of a drug into a target cell described in the aforementioned 1, wherein the average molecular weight of polyethylene glycol of the polyethylene glycol cholesteryl ether is from 200 to 20,000,

3. the formulation for improving delivery of a drug into a target cell described in the aforementioned 1, which further comprises a polyethylene glycol diacylglycerol ester as a part of the liposome-forming agent,
4. the formulation for improving delivery of a drug into a target cell described in the aforementioned 3, wherein the polyethylene glycol diacylglycerol ester is one or two or more species selected from the group consisting of polyethylene glycol distearylglycerol ester, polyethylene glycol dipalmitoylglycerol ester, polyethylene glycol dimyristoylglycerol ester, polyethylene glycol dilaurylglycerol ester and polyethylene glycol dioleylglycerol ester,
5. the formulation for improving delivery of a drug into a target cell described in the aforementioned 3, wherein the average molecular weight of polyethylene glycol of the polyethylene glycol diacylglycerol ester is from 200 to 20,000,

6. the formulation for improving delivery of a drug into a target cell described in the aforementioned 3, wherein molar ratio of the polyethylene glycol cholesteryl ether to the polyethylene glycol diacylglycerol ester is from 1:100 to 100:1,
7. a drug-containing liposome, which comprises a drug, polyethylene glycol cholesteryl ether and a polyethylene glycol diacylglycerol ester, and
8. the drug-containing liposome described in the aforementioned 7, wherein the average particle diameter thereof is 400 nm or less.

In addition, the present invention relates to the use of polyethylene glycol cholesteryl ether for producing a formulation for improving delivery of a drug into a target cell, and relates to the use of the aforementioned polyethylene glycol cholesteryl ether, wherein average molecular weight of polyethylene glycol of the polyethylene glycol cholesteryl ether is from 200 to 20,000. In another embodiment, it further comprises a polyethylene glycol diacylglycerol ester, and it relates to the use of polyethylene glycol cholesteryl ether, wherein the polyethylene glycol diacylglycerol ester is one or two or more species selected from the group consisting of polyethylene glycol distearylglycerol ester, polyethylene glycol dipalmitoylglycerol ester, polyethylene glycol dimyristoylglycerol ester, polyethylene glycol dilaurylglycerol ester and polyethylene glycol dioleylglycerol ester, the use of polyethylene glycol cholesteryl ether, wherein average molecular weight of polyethylene glycol of the polyethylene glycol diacylglycerol ester is from 200 to 20,000, and further the use of polyethylene glycol cholesteryl ether, wherein molar ratio of the polyethylene glycol cholesteryl ether to the polyethylene glycol diacylglycerol ester is from 1:100 to 100:1.

On the other hand, the present invention relates to a method for improving delivery of a drug into a target cell by a liposome, characterized in that polyethylene glycol cholesteryl ether is used as a part of the liposome-forming agent, and it relates to a method for improving delivery of a drug into a target cell by a liposome, wherein average molecular weight of polyethylene glycol of the polyethylene glycol cholesteryl ether is from 200 to 20,000, a method for improving delivery of a drug into a target cell by a liposome, wherein it further comprises a polyethylene glycol diacylglycerol ester, and a method for improving delivery of a drug into a target cell by a liposome, wherein the polyethylene glycol diacylglycerol ester is one or two or more species selected from the group consisting of polyethylene glycol distearylglycerol ester, polyethylene glycol dipalmitoylglycerol ester, polyethylene glycol dimyristoylglycerol ester, polyethylene glycol dilaurylglycerol ester and polyethylene glycol dioleylglycerol ester. In addition, the present invention further relates to the aforementioned improving method, wherein average molecular weight of polyethylene glycol of the polyethylene glycol diacylglycerol ester is from 200 to 20,000, and relates to a method for improving delivery of a drug into a target cell, wherein molar ratio of the polyethylene glycol cholesteryl ether to the polyethylene glycol diacylglycerol ester is from 1:100 to 100:1.

Also, as another side of the present invention, it relates to a drug-containing liposome with improved delivery of a drug into a target cell, which consists of a drug, polyethylene glycol cholesteryl ether and a polyethylene glycol diacylglycerol ester, relates to the aforementioned drug-containing liposome, wherein its average particle diameter is 400 nm or less, further relates to the aforementioned drug-containing liposome, wherein the drug is a cationic drug, and the present invention also relates the aforementioned drug-containing liposome, wherein the drug is an anticancer agent or an antibacterial agent.

The drug according to the present invention means a drug which expresses an onset of pharmacological effect through its interaction with a biological molecule existing in cells. There is no particular limitation as the illustrative drug, but cationic compounds are desirable. This is because they are advantageous in encapsulation into liposomes. Their illustrative examples include doxorubicin, cisplatin, irinotecan, fluorouracil, carmofur, aclarubicin hydrochloride, daunorubicin hydrochloride, doxorubicin hydrochloride, mitomycin, paclitaxel, epirubicin hydrochloride, idarubicin hydrochloride, pirarubicin hydrochloride, bleomycin, peplomycin sulfate, etoposide, irinotecan hydrochloride, nogitecan hydrochloride, vinorelbine tartarate, docetaxel, vincristine sulfate, vindesine sulfate, vinblastine sulfate, tamoxifen citrate, schizophyllan, krestin, gefitinib, cisplatin, cyclophosphamide, thiotepa, and a gene, an antisense, an siRNA and the like nucleic acid medicines. In addition, these are not limited to one drug, and it is possible to use several drugs in combination as occasion demands.

The improved intracellular delivery according to the present invention means to increase amount of a drug delivered into a cell or to increase amount of a compound which reaches the site of action after the compound entered into the cell, and it becomes possible to know the improvement of delivery by various determination methods. For example, in the case of doxorubicin or the like, this can be confirmed by measuring its cell killing effect by the cytotoxicity test shown in the following. Such an effect can be achieved by a liposome prepared with polyethylene glycol cholesteryl ether, preferably further with a polyethylene glycol diacylglycerol ester, as a part of the liposome-forming agent.

The polyethylene glycol cholesteryl ether means a substance in which the hydroxyl group of polyethylene glycol (PEG) or of a PEG derivative and the tertiary hydroxyl group of cholesterol are bonded through ether bond. Average molecular weight (weight average molecular weight) of the PEG moiety of polyethylene glycol cholesteryl ether is preferably within the range of from 200 to 20,000, more preferably within the range of from 400 to 5,000 most preferably within the range of from 1,000 to 2,000. This is because the compound is apt to be released from liposome as the molecular weight of the PEG moiety becomes large in comparison with the cholesteryl ether as the anchor to the liposome, so that the effect cannot be exerted as a result.

The pharmacological effect can be further improved by the use of a polyethylene glycol diacylglycerol ester at the time of forming liposome, in addition to polyethylene glycol cholesteryl ether. As the polyethylene glycol diacylglycerol ester, one or two or more species selected from the group consisting of polyethylene glycol distearylglycerol ester, polyethylene glycol dipalmitoylglycerol ester, polyethylene glycol dimyristoylglycerol ester, polyethylene glycol dilaurylglycerol ester and polyethylene glycol dioleylglycerol ester can be used. Appropriate species are selected based on the other phospholipids constituting the lipid membrane of liposome, kinds of cholesterol, kinds of polyethylene glycol cholesteryl ether and blending ratio thereof. Average molecular weight (weight average molecular weight) of the PEG moiety of polyethylene glycol diacylglycerol ester is preferably within the range of from 200 to 20,000, more preferably within the range of from 400 to 5,000, most preferably within the range of from 1,000 to 2,000.

According to the present invention, molar ratio of the polyethylene glycol cholesteryl ether to the polyethylene glycol diacylglycerol ester is from 1:100 to 100:1, preferably from 1:5 to 5:1, most preferably from 1:2 to 2:1. This is because stability of the liposome itself in blood becomes worse as the blending amount of the polyethylene glycol diacylglycerol ester becomes small, so that its ability to reach the target cell is lost as a result and its in vivo intracellular delivery effect cannot therefore be exerted. Also, this is because the intracellular delivery effect is attenuated when blending amount of the polyethylene glycol cholesteryl ether becomes small.

When the liposome of the present invention is prepared, phospholipids, cholesterol and the like which are generally used in preparing liposomes are blended. The "as a part of the liposome-forming agent" according to the present invention means that the phospholipids, cholesterol and the like as shown in the following can be blended in addition to the polyethylene glycol cholesteryl ether and polyethylene glycol diacylglycerol ester, within such a range that they do not spoil the effect of the present invention. The "liposome-forming agent" according to the present invention means an agent which is necessary when the liposome is prepared. Examples of the phospholipid include egg-yolk lecithin, soybean lecithin, hydrogenated lecithin, distearylphosphatidylcholine, dipalmitoylphosphatidylcholine, dimyristoylphosphatidylcholine, dilaurylphosphatidylcholine, dioleylphosphatidylcholine, distearylphosphatidylglycerol, dipalmitoylphosphatidylglycerol, dimyristoylphosphatidylglycerol, dilaurylphosphatidylglycerol, distearylphosphatidylethanolamine, dipalmitoylphosphatidylethanolamine, dimyristoylphosphatidylethanolamine, dilaurylphosphatidylethanolamine, distearyl phosphatidate, dipalmitoyl phosphatidate, dimyristoyl phosphatidate, dilauryl phosphatidate and the like. It is possible to select one or two or more of them.

Particle diameter of the liposome of the present invention is not particularly limited with the proviso that it is a size that can achieve the effect of the present invention, but it is desirable the average particle diameter is 400 nm or less in the case of its intravenous administration. This is because a liposome having a particle diameter of exceeding 400 nm is cleared by the liver, spleen and the like reticuloendothelial system and the lungs, thus resulting in its short-term retention in blood and small accumulation to the organ where the point of application is present.

The liposome of the present invention is prepared by employing the production methods generally used in the technical field of said liposome preparations. Illustratively, the Bangham method, freeze and thawing method, ethanol injection method or reverse phase evaporation method described in "Liposome, edited by Nojima, published by Nanko-do (1988)", the freeze drying method or pH gradient method described in Methods in Enzymology, 367, pp - 110 (2003) or the like can be employed.

For example, as described in Examples 1 and 2, polyethylene glycol cholesteryl ether, also a polyethylene glycol diacylglycerol ester when necessary, and further an optional liposome-forming agent such as distearylphosphatidylcholine or the like phospholipid are dissolved or suspended in a solvent at a predetermined ratio, and then the solvent is evaporated. This is hydrated by adding a buffer agent and stabilizing agent and subjected to nitrogen replacement, and then a liposome dispersion is prepared by employing an ultrasonic treatment or the like. When the liposome particle diameter is adjusted, an extrusion method or the like is applied thereto.

By employing polyethylene glycol cholesteryl ether, preferably further a polyethylene glycol diacylglycerol ester, the liposome of the present invention with improved delivery to the target cell is provided. Said liposome is administered to the living body by injection, oral intake, inhalation, transnasal administration or other administration method. A filler and the like may be added to such a liposome preparation within such a range that the effect of the present invention is not spoiled. As the filler, pharmaceutically acceptable salts, surfactants, saccharides, amino acids, organic acids, other water-soluble substances and the like can be exemplified.

Illustratively, examples of the salts include potassium L-glutamate, sodium L-glutamate, sodium edetate, sodium caprylate, sodium carbazochromesulfonate, carboxymethylcellulose sodium, sodium citrate, calcium gluconate, sodium gluconate, magnesium gluconate, sodium m-sulfobenzoate, disodium hydrogenphosphate, sodium dihydrogenphosphate, dipotassium phosphate, potassium dihydrogenphosphate, aluminum chloride, calcium chloride, sodium chloride, sodium acetate, sodium carbonate, sodium bicarbonate and the like, examples of the surfactants include sorbitan sesquioleate, sorbitan fatty acid ester, polyoxyethylene(160)polyoxypropylene(30)glycol, polyoxyethylene sorbitan monolaurate, polyoxyethylene castor oil, polyoxyethylene hydrogenated castor oil, polyoxyethylene hydrogenated castor oil 50, polyoxyethylene hydrogenated castor oil 60, Polysorbate 20, Polysorbate 80, Macrogol 400, Macrogol 4000, Macrogol 600 and the like, examples of the saccharides include D-sorbitol, D-mannitol, inositol, xylitol, dextran, glucose, maltose, lactose, sucrose and the like, examples of the amino acids include methionine, aspartic acid, alanine, arginine, glycine, cysteine, taurine, histidine, phenylalanine, glutamic acid, lysine and the like, examples of the organic acid include citric acid, succinic acid, malic acid, lactic acid and the like, and examples of other water-soluble substances include ascorbic acid, human serum albumin, sodium chondroitin sulfate, gelatin, gelatin hydrolysate, heparin sodium and the like.

### Brief Description of the Drawings

Fig. 1 shows results of the cell-killing effects of doxorubicin aqueous solution and doxorubicin liposome preparation upon a P388 leukemia cell.
Fig. 2 shows results of the drug incorporation of doxorubicin aqueous solution and doxorubicin liposome preparation into Ehrlich's ascites carcinoma cell.
Fig. 3 shows results of the antitumor effects (tumor weight) of doxorubicin aqueous solution and doxorubicin liposome preparation in P388 leukemia cell transplanted mice.
Fig. 4 shows results of the tumor doxorubicin concentration of doxorubicin liposome in P388 leukemia cell transplanted mice.

### Best mode for carrying out the invention

The following illustratively describes the present invention with reference to examples, but the scope of the invention is not restricted thereby.

### Examples

### Example 1

A 100 µmol portion of distearylphosphatidylcholine (DSPC), 100 µmol of cholesterol (Chol), 60 µmol of distearylphosphatidylglycerol (DSPG), 15 µmol of a polyethylene glycol cholesteryl ether (PEG-Chol) with its polyethylene glycol moiety having an average molecular weight of 2000 and 18 µmol of doxorubicin were dissolved in a chloroform/methanol mixed solution (4:1, v/v), the organic solvents were evaporated in a reduced pressure under a stream of nitrogen to prepare a lipid film on the inner wall of the flask, and the container was put in a desiccator to completely evaporate the solvents using a vacuum pump. This was mixed with 10 ml of 9.0% sucrose/10 mM lactate buffer (pH 4.0), hydrated at 75°C, subjected to nitrogen replacement and then subjected to an ultrasonication to obtain a liposome dispersion. Thereafter, particle diameter of the liposomes was adjusted by an extrusion method (PEG-CHO(2000)-LDOX).

### Example 2

A 100 µmol portion of DSPC, 100 µmol of Chol, 60 µmol of DSPG, 7.5 µmol of a polyethylene glycol cholesteryl ether (PEG-Chol) with its polyethylene glycol moiety having an average molecular weight of 2000, 7.5 µmol of a polyethylene glycol distearylglycerol ester (PEG-DSG) with its polyethylene glycol moiety having an average molecular weight of 2000, and 18 µmol of doxorubicin were dissolved in a chloroform/methanol mixed solution (4:1, v/v), and then a particle diameter-adjusted liposome dispersion was prepared by the same operation of Example 1 (PEG-(DSG (2000):CHO (2000) = 1:1)-LDOX).

### Example 3

A 100 µmol portion of DSPC, 100 µmol of Chol, 60 µmol of DSPG, 3.75 µmol of a polyethylene glycol cholesteryl ether (PEG-Chol) with its polyethylene glycol moiety having an average molecular weight of 2000, 11.25 µmol of a polyethylene glycol distearylglycerol ester (PEG-DSG) with its polyethylene glycol moiety having an average molecular weight of 2000, and 18 µmol of doxorubicin were dissolved in a chloroform/methanol mixed solution (4:1, v/v), and then a particle diameter-adjusted liposome dispersion was prepared by the same operation of Example 1 (PEG-(DSG(2000):CHO(2000) = 3:1)-LDOX).

### Comparative Example 1

A 100 µmol portion of DSPC, 100 µmol of Chol, 60 µmol of DSPG, and 18 µmol of doxorubicin were dissolved in a chloroform/methanol mixed solution (4:1, v/v), and then a particle diameter-adjusted liposome dispersion was prepared by the same operation of Example 1 (PLDOX).

### Comparative Example 2

A 100 µmol portion of DSPC, 100 µmol of Chol, 60 µmol of DSPG, 15 µmol of a polyethylene glycol distearylglycerol ester (PEG-DSG) with its polyethylene glycol moiety having an average molecular weight of 2000, and 18 µmol of doxorubicin were dissolved in a chloroform/methanol mixed solution (4:1, v/v), and then a particle diameter-adjusted liposome dispersion was prepared by the same operation of Example 1 (PEG-DSG(2000)-LDOX).

### Comparative Example 3

A 18 µmol portion of doxorubicin was dissolved in 10 ml of 9.0% sucrose/10 mM lactate buffer (pH 4.0) and used as a doxorubicin aqueous solution (DOX sol).

### Test Example 1

The preparations prepared in Examples 1 and 2 and Comparative Examples 1, 2 and 3 were adjusted to various concentrations, and the resulting liquids were added to Ehrlich's ascites carcinoma cell (1 x 10⁶ cells) to examine their cell-killing effects using WST.
Based on the results of plotting a relationship between the doxorubicin concentration in each preparation and its cell-killing effect, the doxorubicin concentration by which 50% of cell-killing effect is obtained was calculated (Table 1). By this, it was shown that the liposome preparations of the present invention show significant pharmacological effect in comparison with Comparative Examples.

**Table 1 Cell-killing effect of doxorubicin aqueous solution and doxorubicin liposome preparation upon Ehrlich's ascites carcinoma cell**

| | IC₅₀ (µM) |
|---|---|
| Comparative Example 3 | 3.45 |
| Comparative Example 1 | 6.30 |
| Comparative Example 2 | 2.85 |
| Example 1 | 1.67 |
| Example 2 | 0.60 |

### Test Example 2

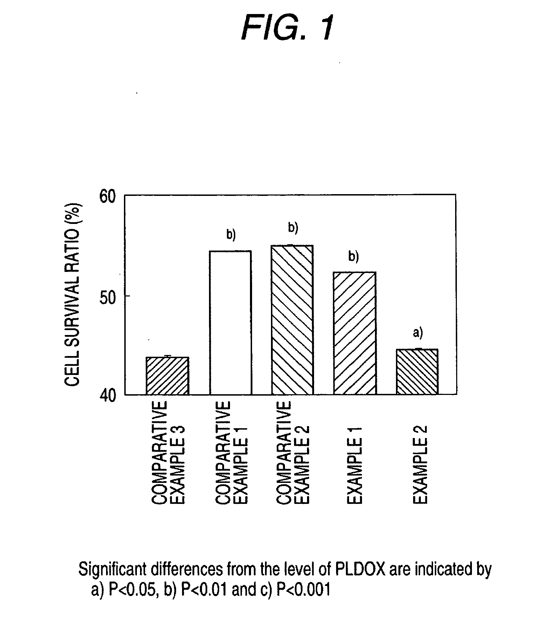

Each of the preparations prepared in Examples 1 and 2 and Comparative Examples 1, 2 and 3 was adjusted to 0.01 µg/ml as the concentration of doxorubicin, and the resulting liquid was added to a P388 leukemia cell (1 x 10⁶ cells) to examine their cell-killing effects using WST in the same manner as in Test Example 1. Ratio of the number of survival cells is shown in Fig. 1.
The liposome preparations of the present invention show significant pharmacological effect in comparison with Comparative Examples 1 and 2 and showed similar effect of the doxorubicin aqueous solution of Comparative Example 3 used as a positive control.

### Test Example 3

The preparations prepared in Examples 1, 2 and 3 and Comparative Example 2 were adjusted to various concentrations, and the resulting liquids were added to a P388 leukemia cell (1 x 10⁶ cells) to examine their cell-killing effects using WST.
Based on the results of plotting a relationship between the doxorubicin concentration in each preparation and its cell-killing effect, the doxorubicin concentration by which 50% of cell-killing effect is obtained was calculated (Table 2). By this, it was shown that the liposome preparations of the present invention show significant pharmacological effect in comparison with the Comparative Example.

**Table 2 Cell-killing effect of doxorubicin aqueous solution and doxorubicin liposome preparation upon P388 leukemia cell**

| | IC₅₀ (µM) |
|---|---|
| Comparative Example 2 | 34.66 |
| Example 1 | 15.61 |
| Example 2 | 12.30 |
| Example 3 | 13.04 |

### Test Example 4

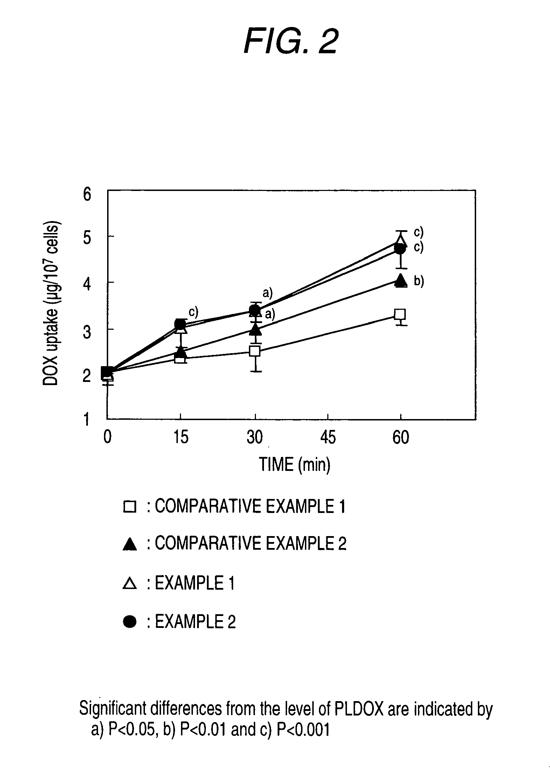

Each of the preparations prepared in Examples 1 and 2 and Comparative Examples 1 and 2 was adjusted to a doxorubicin concentration of 5.0 µg/ml, and the resulting liquid was added to Ehrlich's ascites carcinoma cell, and periodical incorporation of the drug into cells was examined.
Based on the examination of periodical changes in the doxorubicin concentration per the predetermined number of cells, it was shown that significant drug incorporation is obtained when the liposome preparations of the present invention are used, in comparison with those of Comparative Examples (Fig. 2).

### Test Example 5

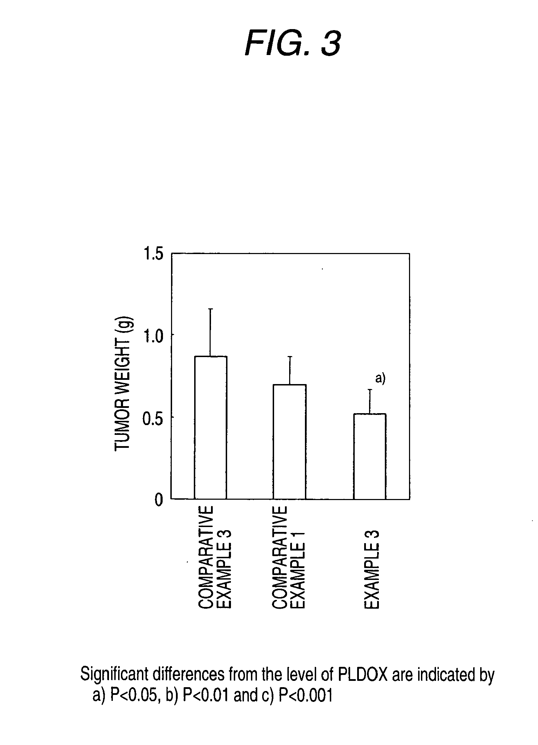

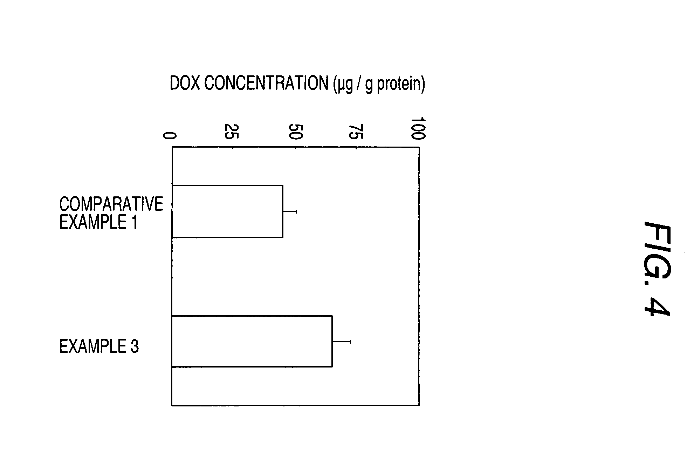

The P388 leukemia cell (5.0 x 10⁵ cells) was inoculated under the dorsal skin of mice to induce a solid tumor. On the 5th, 8th and 11th days after the inoculation, a control (physiological saline) or 2.5 mg/kg, as per dosing of doxorubicin, of each of the preparations prepared in Comparative Example 1 and R Example 3 was injected through the tail vein (total dose 7.5 mg/kg). On the 13th day after the inoculation (48 hours after the third administration), each animal was killed to extract the tumor, and tumor weight and doxorubicin concentration in the tumor cells were measured. The tumor weight was significantly decreased in Example 3 (Fig. 3), and the doxorubicin concentration in the tumor was also increased (Fig. 4).

### Industrial Applicability

The present invention is useful as a provider of a formulation for improving delivery of a drug into a target cell, in which intracellular delivery of the drug by a liposome in the target cell was markedly improved while keeping its retention in the blood circulation without requiring a special operation such as administration of a releasing agent, and of said liposome.

## Claims

1. An formulation for improving delivery of a drug into a target cell, which comprises a liposome prepared using polyethylene glycol cholesteryl ether as a part of the liposome-forming agent.

2. The formulation for improving delivery of a drug into a target cell described in claim 1, wherein the average molecular weight of polyethylene glycol of the polyethylene glycol cholesteryl ether is from 200 to 20,000.

3. The formulation for improving delivery of a drug into a target cell described in claim 1, which further comprises a polyethylene glycol diacylglycerol ester as a part of the liposome-forming agent.

4. The formulation for improving delivery of a drug into a target cell described in claim 3, wherein the polyethylene glycol diacylglycerol ester is one or two or more species selected from the group consisting of polyethylene glycol distearylglycerol ester, polyethylene glycol dipalmitoylglycerol ester, polyethylene glycol dimyristoylglycerol ester, polyethylene glycol dilaurylglycerol ester and polyethylene glycol dioleylglycerol ester.

5. The formulation for improving delivery of a drug into a target cell described in claim 3, wherein the average molecular weight of polyethylene glycol of the polyethylene glycol diacylglycerol ester is from 200 to 20,000.

6. The formulation for improving delivery of a drug into a target cell described in claim 3, wherein molar ratio of the polyethylene glycol cholesteryl ether to the polyethylene glycol diacylglycerol ester is from 1:100 to 100:1.

7. A drug-containing liposome, which comprises a drug, polyethylene glycol cholesteryl ether and a polyethylene glycol diacylglycerol ester.

8. The drug-containing liposome described in claim 7, wherein the average particle diameter thereof is 400 nm or less.
